**Europäisches Patentamt**

**European Patent Office**

**Office européen des brevets**

(11) Publication number: **0 140 854 A2**

(12) **EUROPEAN PATENT APPLICATION**

(21) Application number: **84850292.8**

(22) Date of filing: **01.10.84**

(51) Int. Cl.⁴: **A 61 L 29/00, C 08 J 7/04, C 08 J 7/16, C 09 D 5/16**

---

(30) Priority: **04.10.83 SE 8305444**

(71) Applicant: **ALFA-LAVAL AGRI INTERNATIONAL AB, P O Box 39, S-147 00 Tumba (SE)**
Applicant: **VIGGO AB, P O Box 631, S-251 06 Helsingborg (SE)**

(43) Date of publication of application: **08.05.85 Bulletin 85/19**

(72) Inventor: **Hjerten, Vilhelm Einar Stellan, Langvägen 7B, S-752 52 Uppsala (SE)**
Inventor: **Wadström, Torkel Mikael, P O Box 96, S-741 00 Knivsta (SE)**

(84) Designated Contracting States: **AT BE CH DE FR GB IT LI NL**

(74) Representative: **Inger, Lars Ulf Bosson, L + U INGER Patentbyra HB Garvaregatan 12, S-262 00 Ängelholm (SE)**

---

(54) **Bacteria repellent surfaces.**

(57) The present invention relates to the use of hydrophilic surfaces in order to restrict the adhesion of bacteria on objects coming into contact with bacteria, such as objects within the food stuff industry, and within health care. The hydrophilic surface can be neutral or charged for electrostatic repulsion of bacteria present and/or proteins.

1

BACTERIA REPELLENT SURFACES

DESCRIPTION

Technical field

The present invention relates to the use of hydrophilic neutral or charges surfaces on plastics materials, glass, rubber or metals in order to provide bacteria repellent properties to the surfaces.

The object of the present invention is to obtain a possibility to reduce the number of bacteria and proteins on different surfaces, which are used particularly within the food-stuff industry and within health care.

Background of the invention

It is known from the food-stuff technology that apparatuses comprising details of plastics, glass, rubber, and metals are easily contaminated by bacteria of different types, and need thus be cleaned very often in order to eliminate these. The bacterias, even if not pathogenic, means a hygienic risk and contributes to destruction of the food-stuff being handled in the apparatuses.

The cleansing can often be complicated as the apparatus have to be demounted to a great extent, and means, thereby, and due to operation stand still a great cost. The security at cleansing is, at food-stuff industries, very good, as the whole environment is a low bacteria environment to a large extent. However, within other areas of the handling of food-stuffs environments are present containing high numbers of bacteria, whereby the farming sector is particularly stressed, where one, e.g., at milking has a very bacteria rich millieu.

Thus a far reaching cleansing of apparatuses and pipes and tubes, and cooling of the milk is needed in order to maintain an acceptable low bacterial level. At cleansing strong cleansing agents are used. To clean pipes and tubes, specific pulsation washing methods have been developed, which as such are well-

2

functioning, but as a consequence means energy consumption. It is also important that the milk is rapidly cooled to reduce the growth of bacteria, particularly acidifying Lactobacillus-strains.

It is further known from the health care sector that many patients are caught by different infections after treatments involving introduction of catheters of different types into body ducts, urethra, and urine ducts from the kidneys. Thus it is almost certain that a patient having been treated by means of a urinary catheter and/or Foley-catheter obtains a urinary tract infection, which means a prolonged time of treatment with accompanying increased costs for the society, and the patient. Urinary tract infections are caused by bacteria, which are present at the front, lower opening of the urethra, and in the environment of this opening, which bacteria are attached onto the surface of the catheter, and are brought by means of this up to the urine bladder in spite of general care and carefulness.

Different authors have also studied the problems with bacteria adhesion scientifically and described them. Cf. T J Harris et al Appl. Environ. Microbiol. Vol. 45/3, p. 1018-1024, (1983); and D Rotrosen et al, Jour. Infect. Diseases, Vol. 147/3, p. 594, (1983).

It is also known from US-A-3 695 921 that catheters create infections by mechanical damages, and that an antibiotic compound can be introduced into a polymer to avoid infections.

It is further known from NO-A-149 096 that soft contact lenses have to be sterilized daily, or at least very often to minimize the risk for infections. Such hydrogel lenses are thereby said to provide for growth of patogenic bacteria and fungi thereon.

It has thus been requested surfaces that eliminates, or at least, substantially reduces the adhesion (contamination) of bacteria, and then of course pathogenic bacteria on different materials being used within food-stuff industry and within health care.

Description of the present invention

It has now surprisingly been shown possible to be able to meet thsi requirement by means of the present invention, which is characterized in that a surface being in contact with bacteria and/or viruses have a hydrophilic character.

Further characteristics are evident from the accompanying claims.

Hydrophilic character is a physio-chemical antipode to hydrophobic character. At hydrophobic character it is obtained that non-polar regions of a molecule and non-polar regions of a bed attract each other. Close to the surface of the hydrophobic regions the water present has a higher degree of order than the surrounding water has. If the hydrophobic groups/regions of a molecule/particle, and a bed, respectively, come closer each other the water in order will be pressed aside, and obtains the order of the surrounding, less ordered water, which is equivalent to an increase in entrophy ($\Delta S > 0$). As the change of enthalpy at this type of reactions can be neglected the change of thermodynamic potential, $\Delta G$, is about equal to $-T\Delta S$. As $\Delta S > 0$, $\Delta G$ will be $< 0$, that is that the inter action between the two non-polar regions/groups is a spontaneous reaction. Most biological cells or particles have one or more hydrophobic centre or region.

According to a preferred embodiment of the invention the hydrophilic surface shall have the same charge as the bacterium or other biological compound one wants to repell, i.e., in most cases a negative charge, as most bacteria are negatively charged. The electrostatic repulsion contributes to a further decrease of the tendency of adhesion. Normally, however, the hydrophilic surface shall be neutral, but preferably it shall not have such a charge that electrostatic attraction is obtained.

The present invention will be described more in detail in the

0140854

4

following with reference to some tests carried out.

<u>Tests</u>
Different polymeric materials were tested with regard to bacterial adhesion. Some materials were also tested after modification in order to obtain a hydrophilic character. Samples, 5x5 mm of the different polymeric materials were very carefully cleansed, and were finally rinsed with distilled water and washed with methanol. Thereupon, they were sterilized using a $^{60}$Co-$\gamma$-source (activity: 1600 Ci) with a dose of 2.5 Mrad. The polymeric material samples thus treated were then incubated with a 250 $\mu$l bacterial suspension ($10^8$ cells per ml) at $20^\circ$C. After an incubation period of 1 hr the samples were rinsed using a phosphate buffer in order to eliminate loosely attached or completely free bacteria. The number of bacteria, which were attached to the polymeric materials was measured using a microscope, and luminiscense technology. The water contact angle; the free surface energy ($\gamma sv$); and polar addition to the the free surface energy ($\gamma_s^p$) of the polymeric materials were determined and are given in Table 1 below. Different types of bacteria, particularly patogenic strains of Staphylococcus epidermis, were tested, whereby the bacteria were divided into bacteria having strong hydrophobic character, and bacteria having weaker hydrophobic character.

In the Table 1 below the polymeric materials tested were divided into three groups, viz: a first group comprising commersially available polymers used in the production of medical articles, such as catheters; a second group comprising two surface modified polyurethanes to which a hydrophilic monomer - hydroxyethyl methakrylate (HEMA) - has been crosslinked; and a third group comprising polyvinylidene fluoride, which was surface modified by crosslinking with akrylic acid and then treatment with KOH, whereby a negative charge on the surface was obtained. The third group further comprises glass having been modified with carboxy methyl cellulose (CMC) and a strong hydrophilic basic protein (lysozyme from hen egg white).

Table 1

| Polymer | Contact angle (water-air) ($^\circ$) | Free surface energy ($mN.m^{-1}$) | Polar addition to free surface energy ($mN.m^{-1}$) | Number of attached bacteria $\cdot 10^3$ per $cm^2$ | |
|---|---|---|---|---|---|
| | | | | Strong hydrophobic strains K 11,K 12, Gloor 1/1 | Less strong hydrophobic strains KH 6,Gloor 99 |
| Silicon rubber | 109 | 21 | 0 | $158^\pm 81$ | $55^\pm 33$ |
| Polyetherurethane | 107 | 25 | 2 | $146^\pm 102$ | $60^\pm 25$ |
| Polypropylene (amorphous) | 104 | 30 | 0 | $426^\pm 184$ | $21^\pm 14$ |
| Polyethylene | 95 | 32 | 0-2 | $310^\pm 165$ | $34^\pm 8$ |
| Polyethylene terephtalate | 78 | 40 | 3 | $288^\pm 141$ | $31^\pm 3$ |
| Polyvinylidene fluoride | 77 | 33 | 6 | $348^\pm 152$ | $34^\pm 4$ |
| Celluloseacetate | 58 | 45 | 10 | $118^\pm 72$ | $58^\pm 20$ |
| Polyetherurethane mod. w. HEMA 20% | 88 | 47 | 20 | $26^\pm 4$ | $7.0^\pm 2.3$ |
| Polyetherurethane mod. w. HEMA 130% | 72 | 64 | 30 | $27^\pm 9$ | $7.0^\pm 3.1$ |
| Polyvinylidene fluoride, cross-linked, akrylic acid, treat. w. KOH | 0 | -- | 50 | $6.3^\pm 2.2$ | $2.6^\pm 1.5$ |
| Glass, modified CMC, lysozyme | 0 | 170 | -- | $5.6^\pm 3.4$ | $3.75^\pm 2.3$ |

In a further test polyethylene was compared with polyethylene coated with lysozyme and ployethylene coated with crude slime. The results obtained are given in relative values using the number of bacteria attached to polyethylene as the value 100, and the other in relation hereto. The charge of the active surface is also given.

Table 2

| Polymer | Contact angle ($^{o}$) | Charge | Adhesion rel. value | |
|---|---|---|---|---|
| | | | KH 11 | KH 6 |
| Polyethylene | 95 | $\pm$ | 100 | 100 |
| Polyethylene/ lysozyme | 0 | $\pm$ | 54 | 40 |
| Polyethylene/ crude slime | 0 | − | 19 | 22 |

As evident from the above the polar addition to free surface energy ($\gamma_s^p$) of the hydrophilic surface should be at least 15 mN.m$^{-1}$, preferably at least 20 mN.m$^{-1}$, and the whole character of the hydrophilic surface being such that not more than $50.10^3$ bacteria per cm$^2$ are attached, when strongly hydrophobic bacteria strains are at hand, (Staphylococcus epidermis strain KH 11, Gloor 1/1 are such strains), and not more than $10.10^3$ bacteria per cm$^2$, when less strong hydrophobic strains are at hand, (Staph. epidermis strain KH 6, Gloor 99).

Different types of hydrophilic surfaces can be used. Thus a polymer material can be used for the production of polymer objects, which polymer material is treated with crosslinking monomers to the formation of a hydrophilic surface, such as polyvinylidene fluoride treated with akrylic acid.

Further, polymers, glass, rubber, or metals can be coated with a compound having hydrophilic character, such as e.g. a very high molecular polyoxyethylene compound (Mw >10$^6$), carboxy methyl cellulose (CMC), or dextrane or polyacrylamide.

7

A completely hydrophilic material can be produced from hydrophilic monomers, such as hydroxy lower alkyl acrylate, or methacrylate, hydroxy lower alkoxy lower alkyl acrylate, or methacrylate, such as 2-hydroxy ethylacrylate, 2-hydroxy ethylmethacrylate, diethyleneglycol monoacrylate, diethyleneglycol monomethacrylate, 2-hydroxypropylacrylate, 2-hydroxypropyl methacrylate, 3-hydroxypropyl acrylate, 3-hydroxypropyl methacrylate, and dipropylglycol monomethacrylate. Preferred polymers are hereby hydroxyalkyl acrylates and methacrylates having 2 to 3 carbon atoms in the hydroxyalkyl group. The polymers used shall be waterinsoluble.

Said hydroxyalkyl acrylates and methacrylates can be replaced partly or completely, by vinylpyrrolidone, acrylamide, metharylamide, N-propylacrylamide, N-isopropylmethaorylamide, N-methylacrylamide, N-methylmethacrylamide, N-methylolacrylamide, and N-methylolmethacrylamide, N-2-hydroxyethylacrylamide, and N-2-hydroxyethylmethacrylamide. The last mentioned monomers most often form watersoluble homopolymers, and thus have to be crosslinked using a hydroxyalkyl acrylate, or methacrylate, or another material, which provides waterinsolubility.

Other ethylenically, unsaturated monomers, which can be used together with the above in order to form hydrophilic polymers are acrylonitrile, methacrylonitrile, vinylacetate, alkylacrylates and methacrylates, alkoxyalkylacrylates, and methacrylates. Example of alkylacrylates and methacrylates are methacrylate, ethylacrylate, butylacrylate, 2-ethylhexylacrylate, methylmethacrylate, and butylmethacrylates. Example of alkoxyalkylacrylates and methacrylates are methoxyethylacrylate, methoxyethylmethacrylate, ethoxyethylacrylate, ethoxyethylmethacrylate, propoxyethylacrylate, butoxyethylmethacrylate, methoxypropylacrylate, ethoxypropylmethacrylate. These comonomers are used in an amount of up to 50%, usually 0.5 to 20%. Also other vinylmonomers that contain ionisable, functional groups can be copolymerized using hydroxyalkylacrylates and methacrylates. Thereby acidic monomers, such as acrylic acid, itaconic acid, methacrylic acid, maleic acid, fumaric acid, aconitinic acid, cinnamic acid, crotonic acid, carboxylic acid, propionic acid, citrakonic acid, vinylsulphonic acid, p-vinyl-benzenesulphonic acid, partial esters, such as mono-2-hydroxy-ethylitakonate, mono-2-hydroxypropylcitrakonate, mono-2-hydro-xyethylmaleate, mono-2-hydroxypropylfumarate, monoethylitako-nate, monomethylitakonate, monomethyl cellusolve itakonate, monomethylcellusolve is the monoethyl ether of diethylene glycol), monomethylcellusolve maleate, mono-2-hydroxyethylata-konitate, are included.

Also alkaline monomers are included, such as aminoethylmethacrylate, dimethyl aminoethylmethacrylate, t-butylaminoethylmeth-acrylate, p-aminostyrene, o-aminostyrene, 2-amino-4-vinyltoluene, diethylaminoethyl acrylate, dimethylaminoethylacrylate, t-butyl-aminoethylacrylate, piperidino ethylacrylate, piperidinoethyl methacrylate, morpholinoethyl acrylate, morpholinoethyl methacrylate, 2-vinylpyridine, 3-vinylpyridine, 4-vinylpyridine, 2-ethyl-5-vinylpyridine, dimethylaminopropylacrylate, dimethyl-aminopropylmethacrylate, dipropylaminoethyl acrylate, dimethyl aminoethyl vinylether, dimethylaminoethylvinylsulphide, diethyl-aminoethylvinylether, aminovinylethylether, 2-pyrrolidinoethyl methacrylate, 3-(dimethylaminoethyl)-2-hydroxypropyl acrylate, 3-(dimethylaminoethyl)-2- hydroxypropyl methacrylate, 2-aminoethylacrylate, 2-aminoethyl methacrylate. Alkylaminoethyl acrylates and methacrylates are preferred in this group. Also mul-tipolymers having 3,4 or more monomers can be used.

If the polymer obtained should not be waterinsoluble it can be crosslinked using 1-10 % of a suitable, crosslinking agent, such as ethylene glycol diacrylate, ethyleneglycol dimethacrylate, 1,4-butylenedimethacrylate, diethylene glycol dimethacrylate, propylene glycol dimethacrylate, diethylene glycol dimethacrylate, dipropylene glycol dimethacrylate, diethylene glycol diacrylate, dipropylene glycol diacrylate, divinyl ben-zene, divinyltoluene, diallyl tartrate, allyl puryvate, allyl maleate, divinyl tartrate, triallyl melamine, N,N'-methylene diacrylamide, diallyl maleate, divinylether, diallyl monoethylene glycol citrate, ethylene glycol vinylallyl citrate, allyl vinyl maleate, diallylitakonate, ethylene glycol diester of itakonic acid, divinylsulphone, hexahydro-1,3,5-triacryl triazine, triallyl phosphite, diallyl ether of benzene phosphonic acid, polyester of maleic acid anhydride with triethylene glycol, diallylakonitrate, divinylcitrakonate, diallyl fumarate, ammonium dicromate. Other dicromates are sodium and potassium dicromate.

In order to carry out the polymerization a catalyst in an amount of 0.05 to 1 % be used, such as t-butyl peroctoate, benzoyl peroxide, isopropyl percarbonate, methylethyl ketone peroxide, cumenhydro peroxide, and dicumyl peroxide. Irradiation using UV and gamma rays can also be used. The polymerization can be carried out at 20 to 150°C, usually at 40 to 90°C.

To coat a hydrophobic polymer with a hydrophilic polymer, this is added by means of a solvent, which then is evaporated. The solvent contains preferably crosslinking agents.

Other ways of providing a hydrophilic surface are to treat for example a hydrophobic polymer with a solvent (swelling agent) whereupon a grafting agent such as polyurethane (optionally a prepolymer) comprising a polyisocyanate is attached. To the isocyanate groups a polyvinyl pyrrolidone is then attached at heating. Thus a product of rubber latex can e.g. be treated with dichloromethane, whereupon a polyurethane of the polyestertype dissolved in ethyl acetate is added. The ethyl acetate solution can also contain a prepolymer based on trimethylol propane and diphenyl methane diisocyanate. After some hour the rubber latex product is eliminated from the solution, air dried and is dipped into a solution of polyvinyl pyrrolidone. Hardening is carried out at 65°C after air drying. Further a product of polyether polyurethane be dipped into a solution of diphenylmethane diisocyanate in methyl ethyl ketone, be dried, and then be dipped into a solution of polyvinyl pyrrolidone.

In stead of polyvinylpyrrolidone polyacrylamide can be used, whereby, however, the product is not wear resistant. Polyacrylamide does not attach completely.

Suitable polyurethanes are polytetramethylene ether glycol-diphenyl methane diisocyanate(MDI), polytetramethylene ether glycol-toluene diisocyanate(TDI); polytetramethylene ether glycol-isoferronisocyanate; poly(1,4-oxybutylene)glycol-MDI; poly(1,4-oxybutylene)glycol-TDI; poly(1,4-oxybutylene)isoferronisocyanate; polyethylene glycol-MDI; polyethylene glycol-TDI; polyethylene glycol-isoferronisocyanate; polypropylene glycol-MDI; polypropylen glycol-TDI; polypropylene glycol-isoferronisocyanate;polycaprolactone-MDI; polycaprolactone-TDI; polycaprolactone-isoferronisocyanate; polyethylene adipate-MDI; polyethylene adipate-TDI; polyethylene adipate-isoferronisocyanate; polytetramethylene adipate-MDI; polytetramethylene adipate-TDI; polytetramethylene adipate-isoferronisocyanate; polyethylene propylene adipate-MDI; polyethylene propylene adipate-TDI; polyethylene propylene adipate-isoferronisocyanate.

Other polymers are crosslinked polymers of a polyisocyanate (10 to 95 %) and a copolymer of metylmethacrylate (5 to 70 %), and an olefinic comonomer (0.3 to 10 %) having an isocyanate group, and a group which can react with isocyanate, and an olefinic comonmer (0 to 25 %) having a free isocyanate group.

Further, silicon rubber products can be treated with N-vinylpyrrolidone, which can be polymerized using irradiation to form polyvinylpyrrolidone having hydrophilic properties.

Other hydrophilic produc s are partly or completely copolymers of acrylonitrile with acryl amide or acrylic acid.

In coating objects with polyoxyethylene, this is dissolved in an organic solvent, such as aceton. The concentration of polyoxyethylene is normally 0.5 to 2 % when the molecular weight is 1 to $5.10^6$. The product to be provided with a hydrophilic surface of polyoxyethylene is dipped into the solution and is the allowed to dry.

When treating products with polyethylene or polypropylene surface polyethylene, and polypropylene, respectively, is coupled using acrylamide groups, whereupon, if a charged surface is wanted, one treats with polyethyleneimine to obtain a positive charge, and with sulphta groups to obtain negative charge.

Certain coatings or hydrophilic surfaces are more stable than others, but even more "short-lived" coatings can be useful from a bacterial point of view, if one regards easily, exchangeable simple products of disposable type.

It is evident that most materials within the food stuff industry should be coated with more durable hydrophilic surfaces, while on the other side more simple products used within health care, such as chatheters, sutures, tubes, allows a coating of surfaces which are only stable for a short time period, or which are not particularly stable against mechanical weariness. Mechanical weariness is, as known, rare in most applications within health care.

In the tests carried out above it has also been able to establish that the bacteria, which have attached to hydrophilic surfaces, can easily be washed away using e.g. a sodium chloride solution, or a buffer solution, or other washing liquids, while those bacteria, which have attached to less hydrophilic or to hydrophobic surfaces can not be washed away to any remarkable extent.

Hydrophilic character need not only be obtained using conventional polymers, but can also be obtained using proteins. Thus it has been shown in tests that rubber material, e.g. of the type used in the liners of a teat cup of a milking machine, which after havingbeen treated with albumine obtains such a hydrophilic character that bacterial adhesion of the udder patogenic strain Staph. aureus decreased considerably in comparison with an untreated surface.

12

Glass having been treated with carboxy methyl cellulose and a hydrophilic, alkaline protein (lysozyme) showed also a considerably decreased adhesion of bacteria of marine origine, such as E. coli, different Pseudomonas, and Aeromonas strains.

It is also evident that other surfaces than those mentioned above can be used for hydrophilisation in order to avoid an unwanted growth of bacteria, such as e.g. treatment of marine surfaces to prevent growth of bacteria and algae.

The industrial means for food product-on and/or primary production can consist of articles of polymers, glass, rubber, or metal chosen among these materials that are allowed for use in connection with food. They can have, or can be given a hydrophilic surface as described above in the invention. The articles constitute parts that come into contact with food during its processing in the industry, such as e.g. a dairy plant or on the farm.

In primary production, i.e. milk or meat production on a farm such parts can be the liners of a teat cup, pipes, or tubing for milk transport; milk-touched areas in teat cup claws, in milk coolers, or milk tanks.

In dairy plants, or in the food industry the parts can consist of pipes for transportation, holding and mixing tanks, heat exchangers and so on.

13

CLAIMS

1. The use of hydrophilic surfaces on articles made of polymers, glass, rubber, and metal in order to obtain repulsion of bacteria and/or protein.

2. The use according to claim 1, whereby the articles consist of industrial means for food production or primary production such as of meat and milk.

3. The use according to claim 1, whereby the articles consist of objects used within health care.

4. The use according to claim 1, whereby the articles consist of objects being in contact with marine millieu.

5. The use according to claim 1 or 2, whereby the articles consist of means arranged to come into contact with milk products during handling, inclusive of processing the milk on its way from a milk producing animal to a consumer.

6. The use according to claim 5, whwereby the articles consist of part/parts in equipment for milking.

7. The use according to claim 5, whereby the articles consist of part/parts in equipment for a dairy plant.

8. The use according to claim 1 and 3, whereby the articles consist of products for parenteral, and enteral, respectively, therapy.

9. The use according to claim 8, whereby the article consists of a catheter.

10. The use according to claim 8, whereby the article consists of the extracorporeal part of the catheter.

14

11. The use according to claims 1 and 3, whereby the articles consist of objects for peritoneal dialysis.

12. The use according to claims 1 and 3, whereby the articles consist of implantable materials.

13. The use according to claim 12, whereby the article consists of suture.

14. The use according to one or more of the preceeding claims, whereby the hydrophilic surface is a polyurethane having been modified using hydroxyethyl methacrylate.

15. The use according to one or more of claims 1 to 13, whereby the hydrophilic surface is a polyvinylidene fluoride having been modified using acrylic acid.

16. The use according to one or more of claims 1 to 13, whwereby the hydrophilic surface is polyethylene and/or polypropylene having been modified using acrylic amide.

17. The use according to one or more of the preceeding claims, whereby the hydrophilic surface has an ability of repelling bacteria that the surface after having been incubated for one hour using a bacterial suspension containing $10^8$ bacteria per ml of a strongly hydrophobic bacterial strain does not contain more than $50.10^3$ bacteria per $cm^2$.